# EUROPEAN PATENT SPECIFICATION

(11) **EP 1 129 246 B1**
(45) Date of publication and mention of the grant of the patent: **14.01.2004**
(21) Application number: 99970699.7
(22) Date of filing: 06.10.1999
(51) Int. Cl.: D04H 1/72, D21H 27/00

(54) **A PLANT FOR PRODUCING A WEB-SHAPED PRODUCT OF FIBRES AND POWDER**
VORRICHTUNG ZUR HERSTELLUNG EINES BANDFÖRMIGEN PRODUKTS AUS FASERN UND PULVER
INSTALLATION PERMETTANT DE PRODUIRE UN PRODUIT FIBREUX ET PULVERENT SOUS FORME DE BANDE

(30) Priority: 06.10.1998 DK 126498
(43) Date of publication of application: 05.09.2001
(73) Proprietor: M & J Fibretech A/S, 8700 Horsens (DK)
(72) Inventor: SORENSEN, Elmgaard, Birger, DK-7120 Vejle O (DK)
(74) Representative: Holme, Edvard
(86) International application number: PCT/DK1999/000527
(87) International publication number: WO 2000/023644

(56) References cited:
- EP-A1- 0 678 608
- WO-A1-96/07792
- WO-A1-96/10663
- US-A- 4 640 810
- US-A- 5 432 000
- US-A- 5 471 712

## Description

This invention relates to a plant which, when running, forms - without the use of liquid - an air-laid web of fibres and a powder on a running endless forming wire which is air-permeable and which operates mainly horizontally, and comprising a suction unit positioned under the forming wire, and a forming head positioned above the forming wire; at least one fibre feed duct to supply the forming head with fibres from a fibre source via an air flow; and at least one powder feed duct for by means of another air flow to feed powder from a powder source over the forming wire via a powder distributor.

A powder like a superabsorbent powder, SAP, is very widely used in products which are designed to absorb liquid. Such products include e.g. sanitary napkins, diapers and incontinence products which are all best produced using an air-laid process. During the past few years, it has become usual to add SAP directly during the air-laid process. The procedure is particularly suitable because the forming process takes place with the aid of air only, i.e. without the use of liquid which could inadvertently activate the SAP.

SAP has a higher density than fibre (usually 0.9-1.3) and the same consistency as sugar (particle size 100-800 microns). This explains why, when distributed in a forming head with fibre, SAP remains in the forming head for a much shorter time than the fibre.

Furthermore, since SAP is a powder, it is conventionally blown into the forming head on a jet of air which distributes the powder irregularly and randomly in the forming head.

The accumulated effect of these characteristics is that the distribution of SAP in the finished product is uneven and inhomogeneous.

The applicant's EP patent application no. 94103336.7-2314 describes a forming head with rows of wings which, when running, sweep the fibres across the forming head's perforated base. The rows of wings are positioned slightly at an angle relative to the machine's direction ensuring that the fibres are distributed particularly evenly and uniformly in the material which is laid on the form wire.

It transpires that even with the improved forming head it is impossible to achieve a satisfactorily even and homogeneous distribution of SAP in the finished product. Distribution in the cross section of fibre and SAP will normally be: Fibre +/-3%, SAP +/- 7%.

However, if SAP is to have the optimum effect, it must be distributed precisely and homogeneously in the product.

If this is not the case, the product cannot optimally assimilate the SAP, which means that the product's capacity to absorb liquid is reduced.

From the patent application WO 96/07792 is known a method and a system for producing airlaid paper webs with a contents of powder, e.g. SAP. The system comprises a perforated forming wire and two forming heads mounted above the forming wire. Each forming head receives a flow of air fluidized fibre material through supply channels. The fibre material is distributed over the with of the wire inside the forming heads. A powder dispenser is used for sprinkling down the powder over the entire with of the web delivered from the forming head. Each of said forming heads is a box without bottom. In one embodiment two perforated drums are arranged in the box. In operation, the fibre flow is supplied to the drums which at the same time are rotated such that the fibres are discharged through the perforated walls of the drums. As the powder is sprinkled directly on the forming wire or in the drums it is not possible to obtain a satisfactory even and homogeneous mixing of the powder and fibres as the heavier powder will tend to fall down to the web faster than the lighter fibres.

From WO 96/10663 is known a plant and a process for dry-producing a web-formed product. The plant has three forming heads arranged at top of each other. Each of the forming heads has a perforated bottom which constitutes the ceiling of the underlying forming head, while the perforated bottom of the lower forming head is placed directly over a forming wire. In operation, the powder is in a airflow led into the upper forming head and is from this passing successively to the intermediate forming head through the perforated bottom of this forming head. Cellulose fibres are in another airflow led into the intermediate forming head and the powder and cellulose fibres are from this passing successively through the perforated bottom of this forming head. Thermo-binding fibres are in a third air flow led into the lower forming head and the powder, cellulose fibres and thermo-binding fibres are from this passing successively through the perforated bottom of this forming head onto the forming wire. In this way the powder will not be supplied to a forming head in one airflow and mixed with fibres, which are supplied to the same forming head in another airflow. Therefore, the mixing of the powder and the different fibres in the above named way will not result in a web having a satisfactory even and homogeneous structure. Besides, the powder is blown into the upper forming head in a conventional way causing an uneven distribution of the powder already in this step. Also, it is not possible to produce a web, which has a high concentration of powder as this need to pass at all three perforated bottoms before getting on top of the forming wire.

The object of the invention is to provide a plant of the kind described above which can achieve a more even and more homogeneous distribution of a powder such as SAP, than ever before.

The second object of the invention is to provide a plant of the kind described above which can facilitate a higher concentration of a powder like SAP in the finished product than ever before.

The third object of the invention is to provide a plant of the kind described above which can facilitate placing a powder like SAP at a designated place within the forming head.

What is new and characteristic according to the invention is that a perforated base is placed between the forming wire and the powder distributor, and that the powder distributor is arranged to let the main air flow in the powder duct with its content of powder divides into a number of finer air flows each of which is either blown into or fed directly in the forming head over said perforated bottom. This ensures that the powder is distributed uniformly and evenly in the forming head or a pre-designated area of it.

In a simple and effective embodiment, the powder distributor can be constructed as a pipe-shaped housing with a base, a powder entry duct which is connected to the powder feed duct, and a number of powder exit ducts which communicate with the forming head.

The air flow with its powder content flows from the fibre feed duct into the pipe-shaped housing. On reaching the base, the air stream is divided into a number of finer flows, each of which flows through a separate powder exit duct.

Distribution of the air stream is more even and more uniform if the inside of the base forms a flow body. At the same time, the flow resistance at the transition between the pipe-shaped housing and the powder exits is reduced.

If adjustable vents are fitted inside or in connection with the powder exits, it will be possible to direct the powder to strategic areas of the forming head.

In one possible embodiment the powder distributor is placed outside the forming head and the powder exit ducts are connected to the powder distributor via each their separate air inlets, which advantageously ends beneath the fibre inlet zone in the forming head where the outflowing powder is least disturbed by the fibre air flow.

This being the case, the powder distributor can in the main be positioned vertically with the base uppermost so that the air flow drives the powder upwards, overcoming the force of gravity.

In a second possible embodiment the powder distributor can be placed with the base downwards in the forming head so that the air flow works in the same direction as the force of gravity. In this construction the layout of the powder feed channel is particularly simple and the powder exits can send powder either directly or through short exhaust ducts into the forming head.

The powder can also be distributed linearly directly in the forming head by means of a powder dosing apparatus with a feeder roller which gradually doses powder into a pre-designated area of the perforated base.

When the forming head is of the type which works by means of rows of rotating wings, it can be beneficial for powder dosing to take place immediately above these wings.

When the forming head is of the kind where the fibres are distributed via perforated walls in rotating drums, it can be beneficial for powder dosing to take place in an area close to or inside these drums so that the powder is efficiently mixed with the fibres which are added to the drums.

The invention is explained below. Examples of possible embodiments are described with reference to the drawings, viz.:
Figure 1 is a schematic illustration of a conventional prior art plant with a forming head which forms an air-laid web of fibre and powder,
Figure 2 is a schematic illustration of a first embodiment for a plant according to the invention with a forming head which forms an air-laid web of fibre and powder,
Figure 3 shows the forming head in Figure 2 seen from above,
Figure 4a, b, c, d show a lateral view of four different constructions for inlet ducts for the plant in Figure 2,
Figure 5 shows a lateral cross-section of a powder distributor for the plant shown in Figure 2,
Figure 6 is a schematic illustration of a second embodiment for a plant in accordance with the invention including a forming head which forms an air-laid web of fibre and powder, and
Figure 7 is a schematic illustration of a third embodiment not part of the invention for a plant including a forming head which forms an air-laid web of fibre and powder.

In the following it is assumed that the plant is used to form a web of fibre and superabsorbent powder, SAP, and that the forming head is of the kind which, when running, distributes the fibres and the SAP along the perforated base of the forming head by means of rotating wings.

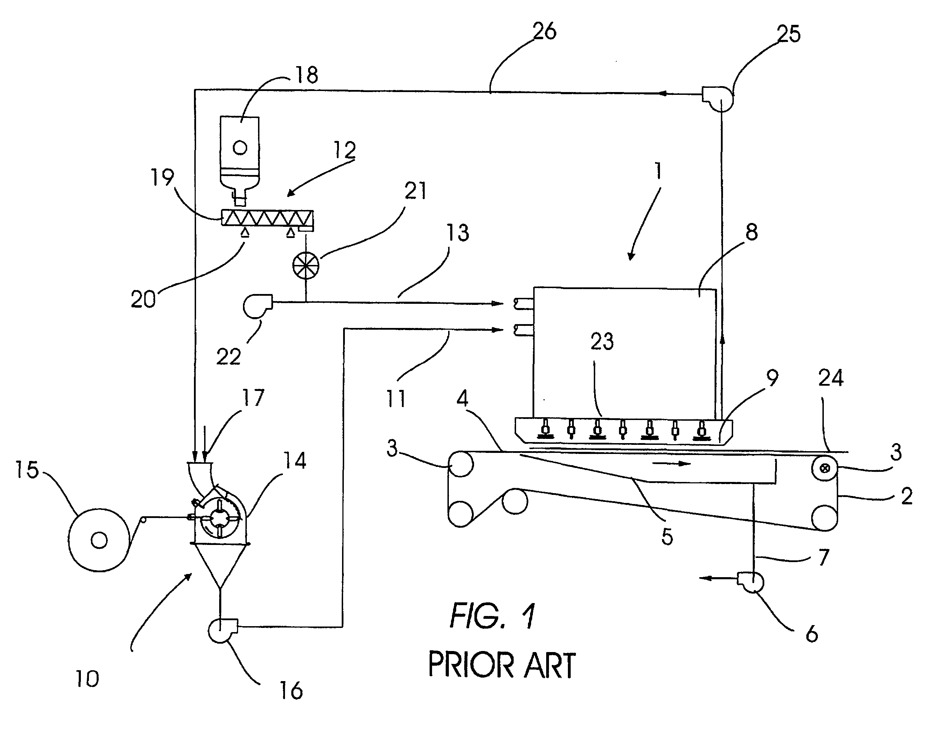

Figure 1 shows a conventional prior art plant which has reference no. 1. The principal components of this plant 1 are an endless air permeable forming wire 2 which - when in operation - runs over the rollers 3; a suction box 5 (mounted under the forming wire's upper part 4) and an adjoined vacuum pump 6 which via suction pipe 7 generate negative pressure in the suction box; a forming head 8 (mounted above the forming wire) with a perforated base 9; a fibre source 10 which is connected to the forming head by a fibre feed duct 11; and a SAP source 12 which is connected to the forming head by a powder feed duct 13.

The fibre source 10 comprises an ordinary defibrator 14 which receives raw material from a fibre stockpile 15. A ventilator 16 sends the defibrated fibres on an air flow to the forming head 8 via the fibre feed channel 11. During this process, air is sucked out of the defibrator and substituted by replacement air via the air pipe 17.

The SAP source 12 comprises a silo 18 with SAP which, when the plant is in operation, is transported on a conveyor 19 on which the desired amount of SAP is continually weighed out by means of weighing cells 20. The SAP is transported via a rotating sluice 21 to a powder feed duct 13 and is blown by means of the ventilator 22 through the powder feed duct into the forming head 8.

The fibre and SAP which, when the plant is running, are blown simultaneously into the forming head, are transported with the help of both gravity and suction from the suction box 5 down towards the base 9 of the forming head and, in the case shown, then pass through seven rows of rotating wings 23 putting the falling material into flowing movements across the forming head's perforated base 9. The material is distributed evenly over the perforated base and simultaneously gradually sucked down through the openings in the base by differential pressure in the forming head 8 and the suction box 5 while new material is fed at the same rate into the forming head via the fibre feed duct 11 and the powder feed duct 13.

Most of the sucked out material then forms a web 24 on the upper side 4 of the forming wire 2, typically in the form of a mesh with a smaller size of meshes than the fibres and the SAP. The upper part of the forming wire 4 runs in the direction indicated by the arrow and transports the web 24 thus formed to the later phases of the process (not shown).

The SAP is blown in something approaching a jet into the forming head, resulting in a very irregular distribution. Furthermore, as the density of SAP is significantly greater than that of fibre, the SAP falls relatively faster down to the base. In addition, the difference in density means that the wings do not distribute the fibres and SAP equally over the base and that the SAP tends to force its way more easily through the openings in the perforated base than the fibre. Under these conditions, the SAP in the web 24 is uneven and inhomogeneous and the quality of the final product is, therefore, not optimum.

During production, nits, i.e. knots in the defibrated material, are formed as a result of imperfect defibration in the defibrator 14, during transport to the forming head 8 or during the processes which take place in it.

These nits reduce the quality of the finished product and are, therefore, extracted from the forming head by a ventilator 25 which is inserted into a nits return duct 26, which connects the back end (in relation to the machine direction) of the forming head 8 to the defibrator 14.

It is to be noted that distribution of fibre in this plant according to the invention is just as efficient in a plant without nits extraction.

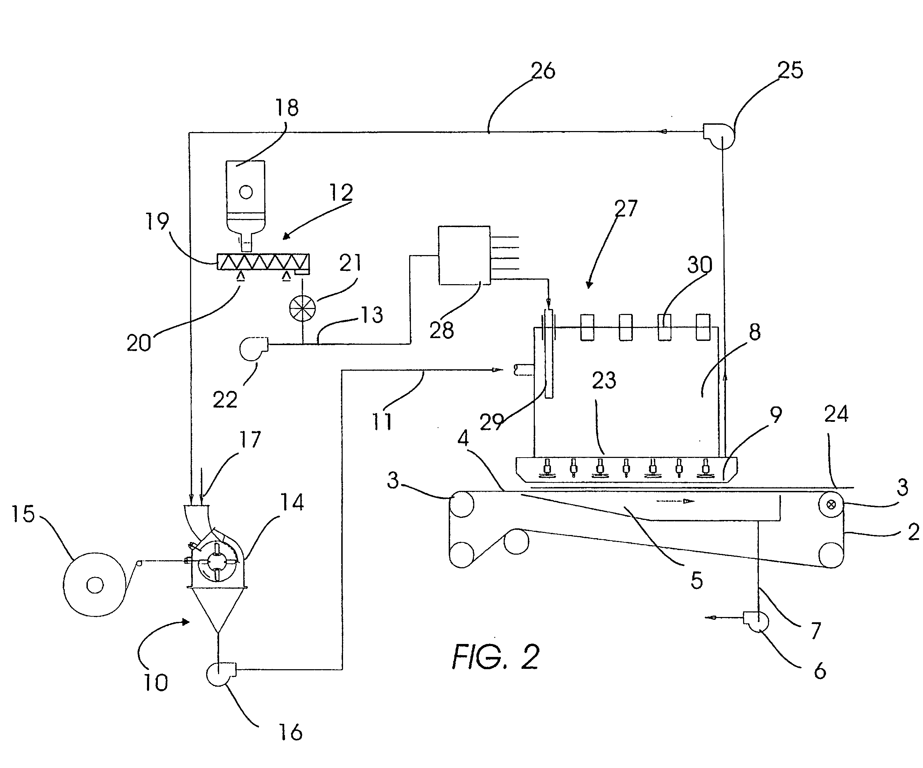

Figure 2 shows a first embodiment of a plant 27 according to the invention. The plant is in the main the same as the conventional plant shown in Figure 1 and identical parts therefore have been given the same reference number.

However, in this case, is a powder distributor 28, which only is illustrated schematically, inserted in the powder feed duct 13. In the powder distributor is the main air flow with its content of powder divided into a number of finer air flows which is blown into the forming head via each their inlet duct 29.

Figure 2 shows only one powder inlet duct. However, there will normally be several inlet ducts, each directed to its own pre-designated strategic area of the forming head.

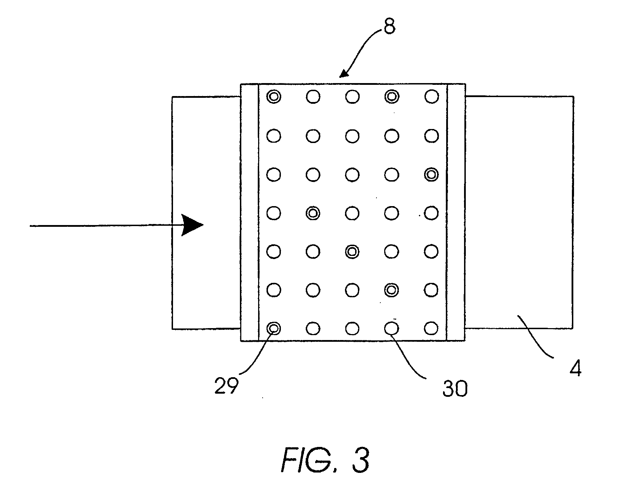

For this purpose the forming head, as shown in Figures 2 and 3, is provided with a number of bushings 30, into each of which a powder inlet duct is inserted. In Figure 3 an inlet duct 29 is inserted down through the bushings (marked with a double ring). The remaining bushings are closed off by a cover (not shown).

In Figure 2 the powder inlet duct 29 empties under the turbulent area where the air from the fibre feed duct 11 blows into the forming head. The fine air flow containing SAP flows out of duct 29 into relatively still air. This ensures that the fine air flow can be directed precisely towards the strategically correct place within the forming head.

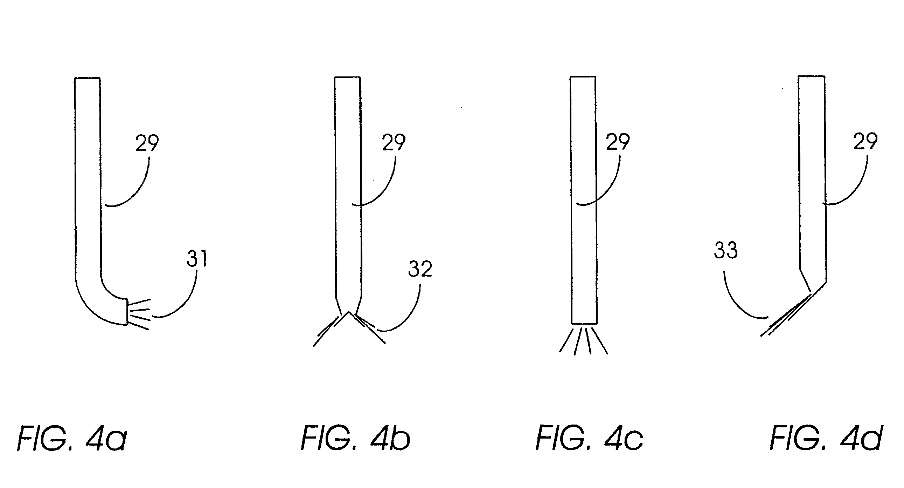

Figures 4a, b, c and d illustrate examples of possible forms for the mouth of the inlet ducts.

In Figure 4a the powder inlet duct 29 has a lower curve 31 which sends the fine air flow containing SAP either horizontally or diagonally into the forming head. This construction is advantageous where an inlet duct is placed close to a wall in the forming head, as the outflowing air will verge away from the wall thus avoiding an uneven distribution of SAP in the forming head. At the same time, the direction of the injection can be adjusted during operation.

In Figure 4b the air flow is divided into two by an angled baffle plate 32. If the inlet duct is placed directly above a row of the rotating wings 23, the SAP blows directly into the materials flowing over the perforated base 9 on both sides of the row of wings concerned.

The mouth shown in Figure 4c is suitable for blowing SAP straight down between two rows of wings.

Figure 4d shows a mouth form which, in this particular case, is equipped with a diagonal baffle plate 33, for in a concentrated jet leading the fine air flow towards an area of the perforated base where a specific amount of SAP is to be added to the material flowing over the base. The direction of the jet of air can be adjusted while the machine is running, i.e. the jet can be directed towards other areas.

In some or all of the inlet ducts there is a valve for regulating the volume flow rate through the respective inlet ducts. The valve will usually be a damper which can be activated either electromagnetically or with the aid of a hydraulic or pneumatic cylinder. The valves ensure that the pattern of powder-laden air in the forming head can be accurately regulated and constantly adjusted during operation.

As mentioned above, conventionally SAP is blown uncontrolled and unevenly into the forming head which means that the distribution of SAP in the product is more or less random and uneven and the quality of the product thus diminished. Using the above-mentioned powder distribution plant in accordance with the invention, it is now possible to control the incoming flow and position the SAP exactly where required in the product, thus optimising the product's characteristics and raw material composition. The product achieves optimum high quality with an accurate and homogeneous SAP distribution, the product functions more uniformly and consumption of SAP is optimised.

Any suitable technique can be used for splitting the main air flow in the fibre feed duct 11 into finer air flows which are then blown under control via air inlet ducts 29 into the forming head (as described above).

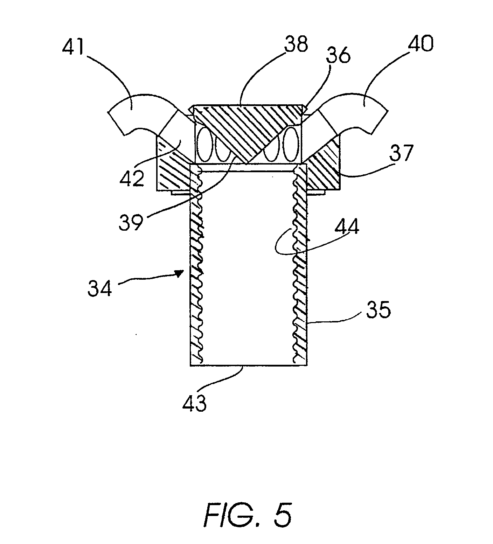

However, Figure 5 is a lateral cross-section illustrating a suitable embodiment of a powder distributor 34. This powder distributor takes the form of a pipe 35 with a hood 36, comprising a skirt 37 which fits tightly around the pipe and a base 38 closing off the end of the pipe. The inside of the pipe is designed as a flow body 39. A number of powder exits 40 extend outwards from the periphery of the powder distributor. Each powder exit 40 comprises a connecting piece 41 and a hole 42 which run diagonally in through the hood's casing close to the flow body 39 at the base.

The open end 43 of the pipe 35 is connected to the fibre feed duct 11 (not shown) and each of the exit connecting pieces 40 is connected to the forming head via its own inlet duct 29 (not shown).

During operation, the main air flow from the fibre feed duct 11 flows into the powder distributor pipe 35. When it meets the base 39, the main air flow is divided into a number of identical finer air flows each of which flows out into a separate powder exit 40 and from there via its own inlet duct into the designated area of the forming head.

The inside surface of the pipe 35 is uneven 44. The uneven surface causes turbulence in the main air flow in the pipe which ensures that the SAP is evenly and homogeneously suspended the air flow so that the finer air flows in the powder exits and inlet ducts contain equal specified quantities of SAP.

In the main the powder distributor's pipe axis is vertical as the force of gravity is neutral relative to the distribution of powder between the various powder exits. Furthermore, the base of the powder distributor may be positioned either outside or within the forming head and with its base either upwards or downwards.

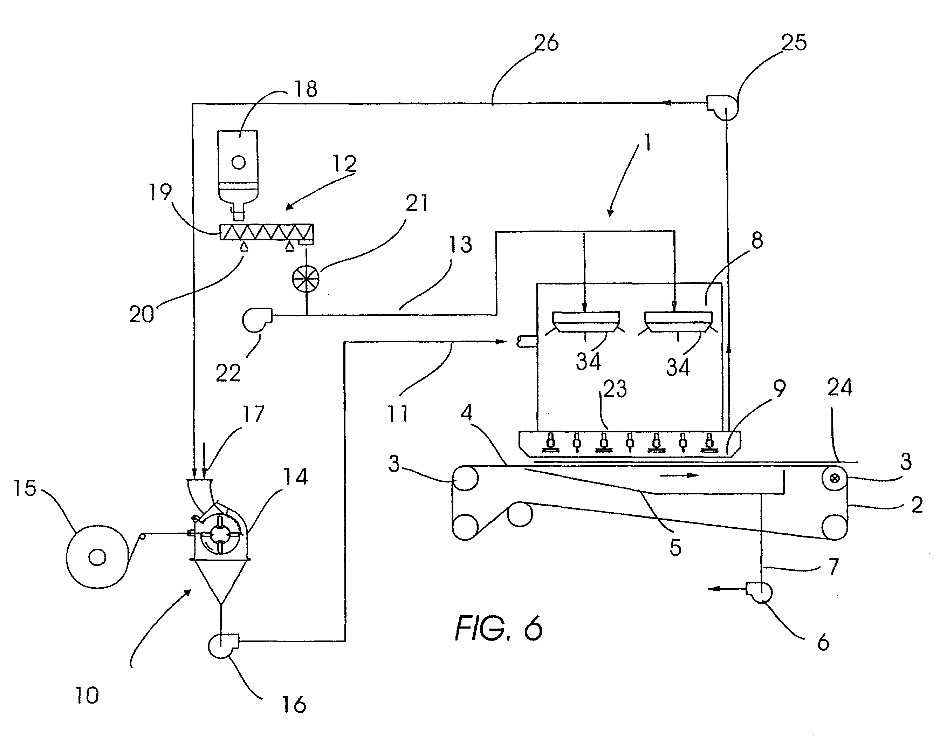

For the most part, the plant shown in Figure 6 corresponds to the plant shown in Figure 2 and identical parts have been given the same reference number. Figure 6 shows a schematic illustration of two powder distributors 34 of the type shown in Figure 5 positioned inside the forming head 8 with their bases downwards. Accommodating the powder distributors within the forming head represents the optimum utilisation of space in the forming head and the connection to the fibre feed duct 11 is simple and efficient. Another benefit is that powder is fed directly in the forming head through the powder exits which may be fitted with short inlet ducts.

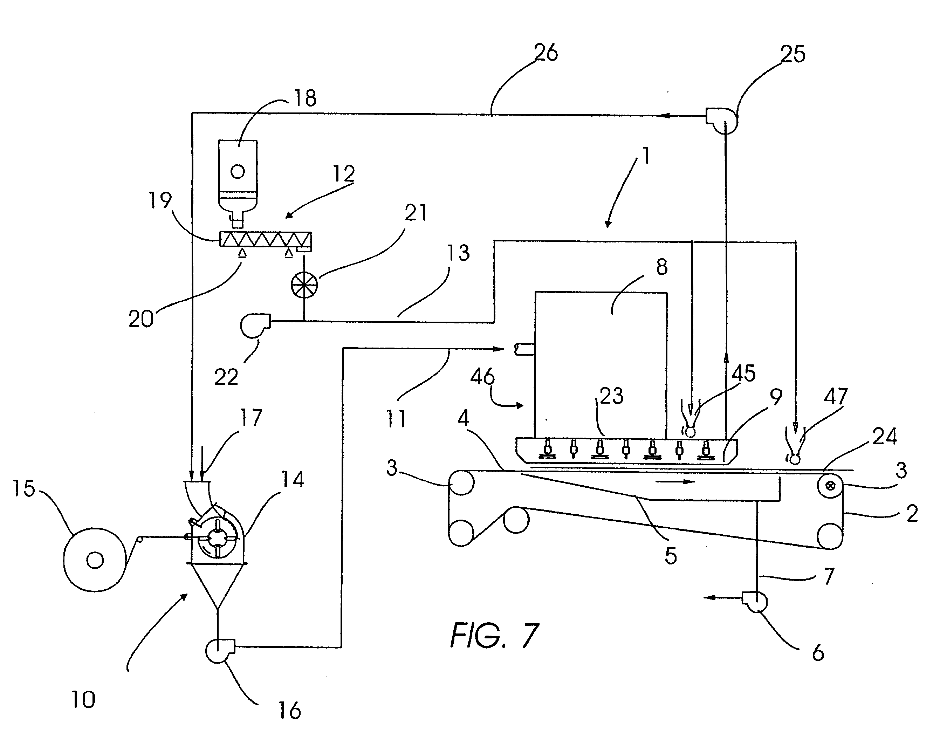

The plant shown in Figure 7 is not part of the present invention. In figure 7 most part corresponds to the plant shown in Figure 2 and identical parts have been given the same reference number. Figure 7 shows a powder distributor in the form of established SAP dosing apparatus 45 which is fitted above an open section of the forming head 46, close to the space between two rows of rotating wings 23 which, in this case, lie at the back end (relative to the machine's direction). A rotating feeding roller, which works like a sowing machine, distributes SAP along a line running at right angles to the machine direction.

This concept ensures a very high degree of accuracy of SAP distribution. In addition, the quantity of SAP transferred to the defibrator causing wear and tear can be reduced.

The SAP dosing apparatus may be mounted either vertically or diagonally and in a sealed section of the forming housing instead of in an open section.

The invention stipulates that the SAP dosing apparatus 45 can be mounted anywhere in the forming housing. The SAP dosing apparatus of figure 7 is not part of the invention, is mounted in a forming head of the kind which distributes fibres and SAP over the perforated base by means of rotating wings and the SAP dosing apparatus can be mounted advantageously in an area close to the area in which the fibres are distributed.

The second SAP dosing apparatus of the plant shown in figure 7, not part of the invention is mounted after the forming head to dose a layer of SAP on top of the web already formed on the forming wire in the area beneath the forming head 46. When a second forming head is mounted to produce a further web over the SAP layer, the resultant product will have an extra concentration of SAP in a central layer which is preferred for many purposes.

There are some air-laid forming heads (not shown) in which fibre is distributed through the perforated walls of rotating drums. In this case, mounting the SAP dosing apparatus in an area very close to or within these drums is beneficial, as this ensures that the SAP is mixed with the fibres in the drums.

In the above, the invention is described assuming that the substance to be distributed in the product is SAP. However, it goes without saying that the plant prescribed in the invention could be used to distribute any other kind of powder or particulate material evenly and homogeneously in a web-formed fibre product.

## Claims

1. Plant (1) which, when running, forms - without the use of liquid - an air-laid web of fibres (24) and a powder on a running endless forming wire which is air-permeable and which operates mainly horizontally, and comprising a suction unit (5) positioned under the forming wire (2), and a forming head (8) positioned above the forming wire (2); at least one fibre feed duct (11) to supply the forming head with fibres from a fibre source (10) via an air flow; and at least one powder feed duct (13) for by means of another air flow to feed powder from a powder source (12) over the forming wire via a powder distributor, **characterised in that** a perforated base (9) is placed between the forming wire and the powder distributor (28;34;45), and that the powder distributor is arranged to let the main air flow in the powder duct (13) with its content of powder divides into a number of finer air flows each of which is either blown into or fed directly in the forming head over said perforated bottom.

2. Plant according to claim 1, **characterised in that** that powder distributor (34) is constructed as a hood-shaped housing with a powder inlet duct (43) which is connected to the powder feed duct (13) and a number of powder exits (41, 42) which communicate with the forming head (8).

3. Plant according to claim 2, **characterised in that** the housing is constructed as a pipe (35) ending close to the powder exits (41,42) in a base (38), that the powder exits (41,42) extend outwards from the periphery of the pipe (35) and that the inside of the base (38) forms a flow body (39) which directs the air flow through the pipe (35) and out into the powder exits (41,42).

4. Plant according to claim 2 or 3, **characterised in that** there is an adjustable valve in at least one of the powder exits (41,42).

5. Plant according to claim 2, 3 or 4, **characterised in that** the powder distributor (28) is mounted outside the forming head (8) and that each powder exit (41,42) is connected to it by a separate inlet duct (29).

6. Plant according to claim 5, **characterised in that** at least some of the separate inlet ducts (29) end beneath the fibre inlet zone in the forming head (8).

7. Plant according to any of the claims 2-6, **characterised in that** at least the powder distributor's (34) powder exits are positioned within the forming head (8).

8. Plant according to any of the claims 2-7, **characterised in that** the powder distributor (34) is in the main vertical with its base (38) downwards and that at least one of the powder exits (41,42) is mounted inside the forming head (8).

9. Plant according to claim 1, **characterised in that** the powder distributor is a powder dosing apparatus (45).

10. Plant according to claim 1 or 2 and where the forming head is of the kind in which the fibres are distributed through perforated walls in rotating drums, **characterised in that** the powder dosing apparatus is positioned in an area close to or inside these drums.

## Patentansprüche

1. Anlage, die, wenn in Betrieb, - ohne Verwendung einer Flüssigkeit - ein luftgelegtes Fasergewirk (24) und ein Pulver auf einem endlos laufenden, luftdurchlässigen Formband, das hauptsächlich horizontal arbeitet, bildet, mit einem unterhalb des Formbandes (2) angeordneten Formkopf (5) und einem oberhalb des Formbandes (2) angeordneten Formkopf (8), wenigstens einem Faserführungskanal (11) zum Versorgen des Formkopfes mit Fasern von einer Faserquelle (10) mittels eines Luftstroms und wenigstens einem Pulverführungskanal (13) zum Zuführen eines Pulvers von einer Pulverquelle (13) über das Formband mittels eines weiteren Luftstroms durch einen Pulververteiler, **dadurch gekennzeichnet, dass** eine perforierte Basis (9) zwischen dem Formband and dem Pulververteiler (28; 34; 45) angeordnet ist und dass der Pulververteiler dazu eingerichtet ist, dass sich der Hauptluftstrom in dem Pulverkanal mit seinem Gehalt an Pulver in eine Anzahl von feinen Luftströmen verteilen kann, die über den perforierten Boden in den Formkopf entweder eingeblasen oder direkt eingeführt werden.

2. Anlage nach Anspruch 1, **dadurch gekennzeichnet, dass** der Pulververteiler (34) als kappenförmiges Gehäuse mit einem Pulvereinlasskanal (43), der mit dem Pulverspeisekanal (13) und einer Anzahl von Pulverauslässen (41, 42), die mit dem Formkopf (8) kommunizieren, verbunden ist.

3. Anlage nach Anspruch 2, **dadurch gekennzeichnet, dass** das Gehäuse als Rohr (35) ausgebildet ist, das nahe den Pulverauslässen (41, 42) in einer Basis (38) endet, dass sie Pulverauslässe (41, 42) sich von dem Umfang des Rohres (35) erstrecken und dass das Innere der Basis (38) einen Stromkörper (39) bildet, der den Luftstrom durch das Rohr (35) und hinaus in die Pulverauslässe (41, 42) richtet.

4. Anlage nach Anspruch 2 oder 3, **dadurch gekennzeichnet, dass** ein einstellbares Ventil in wenigstens einem der Pulverauslässe (41, 42) vorhanden ist.

5. Anlage nach Anspruch 2, 3 oder 4, **dadurch gekennzeichnet, dass** der Pulververteiler (12) außerhalb des Formkopfes (8) montiert ist und dass jeder Pulverausgang (41, 42) mit diesem mittels eines gesonderten Einlasskanals (29) verbunden ist.

6. Anlage nach Anspruch 5, **dadurch gekennzeichnet, dass** wenigstens einige der gesonderten Einlasskanäle (29) unterhalb der Fasereinlasszone in dem Formkopf (8) enden.

7. Anlage nach einem der Ansprüche 2 bis 6, **dadurch gekennzeichnet, dass** wenigstens die Pulverauslässe des Pulververteilers (34) innerhalb des Formkopfs (8) angeordnet sind.

8. Anlage nach einem der Ansprüche 2 bis 7, **dadurch gekennzeichnet, dass** der Pulververteiler (34) in der Hauptvertikalen mit seiner Basis (38) nach unten weist und dass wenigstens einer der Pulverauslässe (41, 42) im Inneren des Formkopfs (8) angeordnet ist.

9. Anlage nach Anspruch 1, **dadurch gekennzeichnet, dass** der Pulververteiler eine Pulverdosiereinrichtung (45) ist.

10. Anlage nach Anspruch 1 oder 2, bei der der Formkopf von der Art ist, bei der die Fasern durch perforierte Wände in Drehtrommeln verteilt werden, **dadurch gekennzeichnet, dass** die Puderdosiervorrichtung in einem Bereich nahe oder im Inneren der Trommel angeordnet ist.

## Revendications

1. Installation (1) qui, lorsqu'elle est en fonctionnement, forme - sans utiliser de liquide - une feuille continue de fibres à couche appliquée par jet d'air (24) et une poudre sur une toile de formage sans fin en mouvement qui est perméable à l'air et qui fonctionne principalement horizontalement, et qui comprend un organe d'aspiration (5) positionné au dessous de la toile de formage (2), et une tête de formage (8) positionnée au dessus de la toile de formage (2) ; et au moins un conduit d'alimentation en fibres (11) destiné à alimenter la tête de formage par des fibres provenant d'une source de fibres (10) par l'intermédiaire d'un flux d'air ; et au moins un conduit d'alimentation en poudre (13) permettant, au moyen d'un autre flux d'air, d'alimenter par une poudre provenant d'une source de poudre (12) la toile de formage par l'intermédiaire d'un distributeur de poudre, **caractérisée en ce qu'**une base perforée (9) est placée entre la toile de formage et le distributeur de poudre (28 ; 34 ; 45), et **en ce que** le distributeur de poudre est agencé de manière à ce que le flux principal d'air pénétrant dans le conduit d'alimentation en poudre (13) avec son contenu de poudre se divise en un certain nombre de flux d'air plus ténus qui sont chacun soufflés ou fournis directement dans la tête de formage disposée au dessus dudit fond perforé.

2. Installation selon la revendication 1, **caractérisée en ce que** le distributeur de poudre (34) est construit comme un réceptacle en forme de capuchon avec un conduit d'entrée de poudre (43) qui est connecté au conduit d'alimentation de poudre (13) et qui comporte un certain nombre de sorties de poudre (41, 42) qui communiquent avec la tête de formage (8).

3. Installation selon la revendication 2, **caractérisée en ce que** le réceptacle est construit comme un tuyau (35) se terminant à proximité des sorties de poudre (41, 42) dans une base (38), **en ce que** les sorties de poudre (41, 42) s'étendent vers l'extérieur depuis le pourtour du tuyau (35) et **en ce que** l'intérieur de la base (38) forme un corps d'orientation de flux (39) qui dirige le flux d'air à travers le tuyau (35) pour qu'il sorte par les sorties de poudre (41, 42).

4. Installation selon la revendication 2 ou 3, **caractérisée en ce qu'**il existe une vanne réglable dans au moins une des sorties de poudre (41, 42).

5. Installation selon la revendication 2, 3 ou 4, **caractérisée en ce que** le distributeur de poudre (28) est monté à l'extérieur de la tête de formage (8) et **en ce que** chaque sortie de poudre (41, 42) est reliée à celui-ci par un conduit d'entrée séparé (29).

6. Installation selon la revendication 5, **caractérisée en ce que** certains au moins des conduits d'entrée séparés (29) se termine au dessous de la zone d'entrée des fibres dans la tête de formage (8).

7. Installation selon l'une quelconque des revendications 2 à 6, **caractérisée en ce que** les sorties de poudre (34) du distributeur de poudre sont au moins positionnées à l'intérieur de la tête de formage (8).

8. Installation selon l'une quelconque des revendications 2 à 7, **caractérisée en ce que** le distributeur de poudre (34) est disposé sur la verticale principale en ayant sa base (38) orientée vers le bas et **en ce qu'**au moins une des sorties de poudre (41, 42) est montée à l'intérieur de la tête de formage (8).

9. Installation selon la revendication 1, **caractérisée en ce que** le distributeur de poudre est un appareil de dosage de poudre (45).

10. Installation selon la revendication 1 ou 2, où la tête de formage est du type dans lequel les fibres sont distribuées par des parois perforées dans des tambours rotatifs, **caractérisée en ce que** l'appareil de dosage de poudre est positionné dans une région située à proximité ou à l'intérieur des tambours.
